# EUROPEAN PATENT SPECIFICATION

(11) **EP 4 294 330 B1**
(45) Date of publication and mention of the grant of the patent: **02.04.2025**
(21) Application number: 22708852.3
(22) Date of filing: 14.02.2022
(51) Int. Cl.: A61F 2/24, A61F 2/962, A61F 2/95

(54) **CATHETER DEVICE FOR DELIVERING A MEDICAL IMPLANT ALLOWING OVERTRAVEL OF A CAPSULE OF THE CATHETER DEVICE**
KATHETERVORRICHTUNG ZUR EINFÜHRUNG EINES MEDIZINISCHEN IMPLANTATS, DIE EINE ÜBERBEWEGUNG EINER KAPSEL DER KATHETERVORRICHTUNG ERLAUBT
DISPOSITIF DE CATHÉTER POUR L'ADMINISTRATION D'UN IMPLANT MÉDICAL PERMETTANT LE DÉPASSEMENT DE COURSE D'UNE CAPSULE DU DISPOSITIF DE CATHÉTER

(30) Priority: 17.02.2021 US 202163150324 P; 09.03.2021 EP 21161475
(43) Date of publication of application: 27.12.2023
(73) Proprietor: BIOTRONIK AG, 8180 Bülach (CH)
(72) Inventor: BURKE, Jeff Alan, Fallbrook, California 92028 (US); DENISON, Andy Edward, Temecula, California 92591 (US); FOERSTER, Markus, 5105 Auenstein (CH); BLANK, Ole, 8006 Zürich (CH)
(74) Representative: Biotronik Corporate Services SE
(86) International application number: PCT/EP2022/053512
(87) International publication number: WO 2022/175202

(56) References cited:
- US-A1- 2014 067 050
- US-A1- 2019 083 261

## Description

The present invention relates to a catheter device for implanting a medical implant, particularly self-expanding implants such as an occluding device, aortic grafts, a prosthetic heart valve, more particularly an aortic heart valve prosthesis (TAVI/TAVR), but also e.g. for an implantable leadless pacemaker.

Such a catheter device usually comprises a capsule for receiving the implant when the latter is in a collapsed state, e.g., a crimped prosthetic heart valve such as a prosthetic aortic heart valve. The capsule covers the implant (e.g. a prosthetic aortic heart valve) that is positioned on a support element connected to an inner sheath of the catheter device while the capsule is connected to an outer sheath (the inner and outer sheaths may also be denoted as inner and outer shafts of the catheter).

Retraction of the outer sheath with respect to the inner sheath allows displacing the capsule with respect to the inner sheath and the support element so as to deploy and release said implant. With the catheter device of the present invention, an implant like a prosthetic aortic heart valve may be, e.g., only partially released and can be retracted into the capsule for the purpose of re-positioning the implant so that it can be deployed at a proper implantation site in a next attempt. Reinserting the implant into the capsule is commonly termed *"resheathing"* and is for example achievable by providing a connection of the implant to a connector connected to the inner sheath of the catheter device.

Catheter devices of the aforementioned kind, particularly for implantation of a self-expanding transcatheter aortic valve replacement (TAVR) prosthesis (or TAVI prosthesis), are challenging in multiple aspects. A typical drawback is the fact that during recapturing of the TAVI/TAVR implant the catheter is exposed to high axial loads. In challenging anatomies or disadvantageous circumstances, these loads can plastically deform the sheaths of the catheter, which can affect a resheathing of the implant to the extend that the capsule can no longer completely cover the implant. However, the withdrawal of the device from the implantation site/body is only considered safe if the capsule is fully closed, i.e., properly aligned with the catheter tip so that no gap is present between the tip and a distal edge of the capsule.

Typically, safety buttons are known in the prior art which are used to limit the uncovering of the prosthesis. Mechanisms of this kind are for instance described in US 9,782,257 B2, US 9,545,308 B2, US 9,615,924 B2, US 8,814,931 B2, US 8,778,019 B2 and US 8,926,693 B2.

US 2014/0067050 A1 describes a prosthesis delivery system comprising a handle assembly with a first control mechanism; a second control mechanism; and a decoupling mechanism; and a delivery catheter with an outer shaft controlled by the first control mechanism; and
a prosthesis containing a capsule.

Based on the above, a problem to be solved by the present invention is to provide a catheter device, particularly a TAVI/TAVR catheter device for a self-expanding aortic valve prosthesis, that allows implanting an implant, particularly a prosthetic heart valve, in particular a self-expanding aortic valve prosthesis, with the ability to allow proper closing of the capsule for the purpose of covering the implant during a resheathing procedure even in case of a permanent compression of the outer sheath and/or a permanent elongation of the inner sheath due to axial loads that have acted on the catheter device during implantation of the implant.

This problem is solved by a catheter device having the features of claim 1. Further embodiments are stated in the sub claims and are also described further below.

According to claim 1, a catheter device for implanting a medical implant is disclosed, wherein the catheter device comprises:
- an outer sheath extending along a longitudinal axis of the catheter device and surrounding a lumen of the outer sheath,
- an inner sheath extending along the longitudinal axis, wherein the inner sheath is arranged in the lumen of the outer sheath and connected to a support element for supporting the medical implant,
- a capsule connected to a distal end of the outer sheath for covering the medical implant when the medical implant is arranged on the support element,
- a handle comprising: a grip portion for manually holding the handle, a rotatable deployment knob, and a traveler, wherein the outer sheath is connected to the traveler, wherein the deployment knob comprises a helical groove formed in an inside of the deployment knob, wherein the traveler engages into the helical groove of the deployment knob such that the traveler and thereby the outer sheath are moved in a proximal direction along the longitudinal axis with respect to the inner sheath when the deployment knob is rotated in a first rotation direction, so that the capsule is pulled away from the medical implant in the proximal direction to deploy the medical implant, and such that the traveler and thereby the outer sheath are moved in the distal direction along the longitudinal axis with respect to the inner sheath when the deployment knob is rotated in an opposite second rotation direction, so that the capsule is moved over the medical implant to cover the medical implant, wherein the handle comprises a locking element that is configured to be manually moved by a user from a first state to a second state, wherein the locking element is configured to limit a movement of the traveler and therewith of the capsule in the distal direction in the first state and wherein the locking element is configured to allow further movement of the traveler and therewith of the capsule in the distal direction in the second state.

During recapturing of the TAVI/TAVR implant, the catheter is exposed to high axial loads. In challenging anatomies or disadvantageous circumstances, these loads can plastically deform the sheaths of the catheter, particularly said inner and outer sheaths. To ensure that the entire implant can still be covered by the capsule, additional travel of the potentially deformed outer sheath is required. Advantageously, this additional travel can be locked and enabled using the locking element. In addition, the mechanism preferably enables automatic resetting of the locking element after use and it may also provide feedback to the user that the locking element has been deactivated or reactivated.

Advantageously, the invention enables a further movement of the capsule in the distal direction so as to compensate the resulting length mismatch between the outer and inner sheath. With this additional travel, that is also denoted as *"overtravel",* the outer sheath can be moved further in the distal direction than its original start position.

Furthermore, in the case that the sheaths do not deform in the procedure, the user could potentially close the capsule too far which would result in a sharp edge on the distal end. Therefore, the present invention comprises a locking element so that the overtravel is only available when the locking element is moved to the second state. The activation and deactivation of the compensation feature is thus safe, detectable and reversible.

In the framework of the present invention, the notion "distal" refers to a portion or components of the catheter device that is remote from the handle or from the physician that operates the catheter device while the notion "proximal" refers to those portions or components that are closer to the handle or closer to the physician.

According to a preferred embodiment of the catheter device according to the present invention, the locking element is configured to set a limit to the movement of the traveler in the distal direction such that the capsule completely covers the implant when the traveler has reached this limit and the inner and the outer sheath have not yet been permanently deformed during usage of the catheter and each comprise their initial length in the direction of the longitudinal axis.

According to a further preferred embodiment, in the second state, the locking element is configured to allow further movement of the traveler and therewith of the capsule in the distal direction such that the implant is completely coverable by the capsule despite a permanent compression of the outer sheath or a permanent elongation of the inner sheath. Without the possibility of said further movement (i.e. overtravel) of the capsule, a gap between the catheter tip and the capsule could not be closed properly so that a resheathing procedure could not be successfully completed or would bear the risk of injuring the patient.

Furthermore, according to an embodiment of the present invention, the handle of the catheter device comprises an elongated rod that is slidable along the longitudinal axis, wherein when the locking element is in its first state, the rod is configured to be pressed by the traveler against the locking element to limit the movement of the traveler (and therefore of the capsule) in the distal direction, wherein a first spring element connected to the traveler is arranged between the traveler and the rod and is pretensioned against the rod when the traveler presses against the locking element.

Preferably, the rod comprises a proximal end portion having a concave face side to enclose a front side of the traveler in a form-fitting fashion when the traveler presses against the rod via said face side of the proximal end portion of the rod.

According to a further embodiment, the locking element is configured to release the rod when the locking element is moved from the first state to the second state, wherein the rod is pushed in the distal direction by means of the pretensioned first spring element that is allowed to expand when the locking element sets the rod free in the second state.

Furthermore, according to an embodiment, when the locking element is in the second state, the further movement (so-called overtravel) of the traveler in the distal direction caused by further rotation of the deployment knob in the first rotation direction pushes the rod against a stop provided on the handle which prevents any further movement of the traveler and therewith of the capsule in the distal direction. Now, the first spring element is pretensioned by the traveler against the rod, and a second spring element is pretensioned by the rod against a guiding member of the handle, which guiding member is configured to guide the sliding movement of the rod along the longitudinal axis and also forms said stop for the rod.

According to a further embodiment of the catheter device, the handle comprises an actuating element configured to be manually actuated by a user, wherein the actuating element is operatively connected to the locking element, wherein the locking element is moved from the first state to the second state when the actuating element is actuated by the user. Particularly, the actuating element can be integrally connected to the locking element. Particularly, the actuating element can be a pushable button, wherein actuating the actuating element corresponds to manually pushing the button which causes the locking element to transition from the first state to the second state.

According to yet another embodiment of the catheter device, the handle comprises a third spring element, wherein the third spring element is configured to be pretensioned against the locking element when the latter is moved from the first state to the second state. The third spring element thus provides a restoring force that allows moving the locking element back from the second state to the first state as will be described further below.

According to a further embodiment, the locking element comprises an opening having a narrow portion and an adjacent wider portion, wherein the rod comprises a distal portion comprising a narrow section having a diameter that is smaller than a diameter of an adjacent proximal portion of the rod (that connects to the proximal end portion of the rod) so that the rod forms a step, particularly a circumferential step, at said narrow section, wherein when the locking element is in the first state said narrow section of the rod is arranged in the narrow portion of the opening of the locking element and the rod is configured to be pressed by the traveler against the locking element with said step to prevent the movement of the traveler in the distal direction.

According to a further embodiment, when the locking element is moved from the first state to the second state, the rod becomes arranged in the wider portion of the opening of the locking element so that the step of the rod is allowed to move through the wider portion of the opening of the rod by the action of the pretensioned first spring element when the latter expands.

The deactivated locking element that allows movement of the rod in the distal direction in the second state can be automatically transferred back into the first state using the pretensioned third spring element when the capsule is opened again, i.e. by moving the outer sheath via the traveler in the proximal direction.

Particularly, when the traveler pushes the rod against the stop (which means that the maximal overtravel of the capsule has been realized) or when the second spring element is pretensioned, moving the traveler and therewith the capsule in the proximal direction (to again deploy the implant) causes the pretensioned second spring element to move the rod in the proximal direction, too, such that said narrow section of the rod moves back into the opening of the locking element at a certain position of the traveler which allows the third spring element to move the locking element back into the first state in which said section of the rod is in turn arranged in the narrow portion of the opening of the locking element.

The locking element has now returned to the first state and will limit further movement of the capsule in the distal direction until it is again moved to the second state by manually actuating, e.g. pushing, the actuating element (e.g. pushable button).

According to a further embodiment of the present invention, the deployment knob is rotatably supported on the guiding member of the handle that forms the stop for the rod and is also configured to guide the movement of the rod along the longitudinal axis.

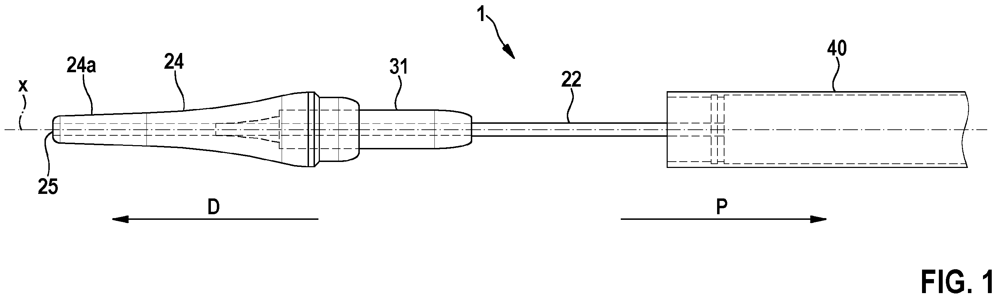

In the following, further features and advantages of the present invention are described in detail with reference to the Figures which show preferred embodiments of the catheter device according to the present invention, wherein
- Fig. 1: shows a distal portion of an embodiment of a catheter device according to the present invention comprising a catheter tip and a capsule for covering the implant such that a distal end of the capsule is properly aligned with the catheter tip so that the capsule is arranged flush with the catheter tip and no portion of the implant sticks out of the capsule;

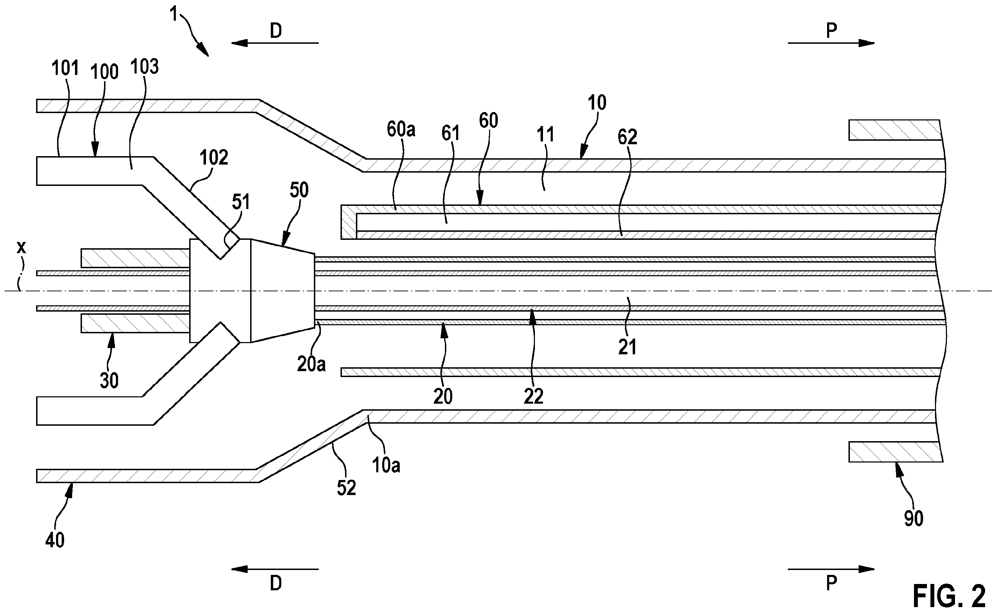

- Fig. 2: shows a schematic cross sectional view of a portion of an embodiment of a catheter device according to the present invention, wherein the medical implant is covered by the capsule and connected to a connector at a distal end of the inner sheath; also shown is a stabilizing sheath that is connected to the grip portion of the handle (stabilizer) and a deflection sheath for adjusting the angular position of the capsule/medical implant;

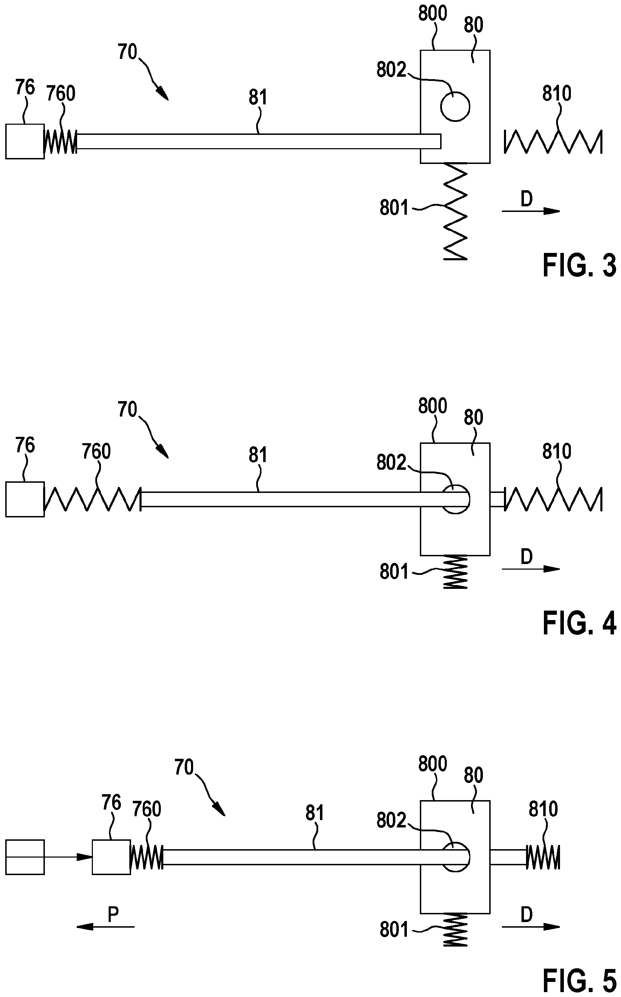

- Figs. 3 to 5: show schematical illustrations of a traveler of an embodiment of a catheter device according to the present invention and its interaction with a locking element for limiting/allowing a further movement of the capsule of the catheter device in the distal direction;

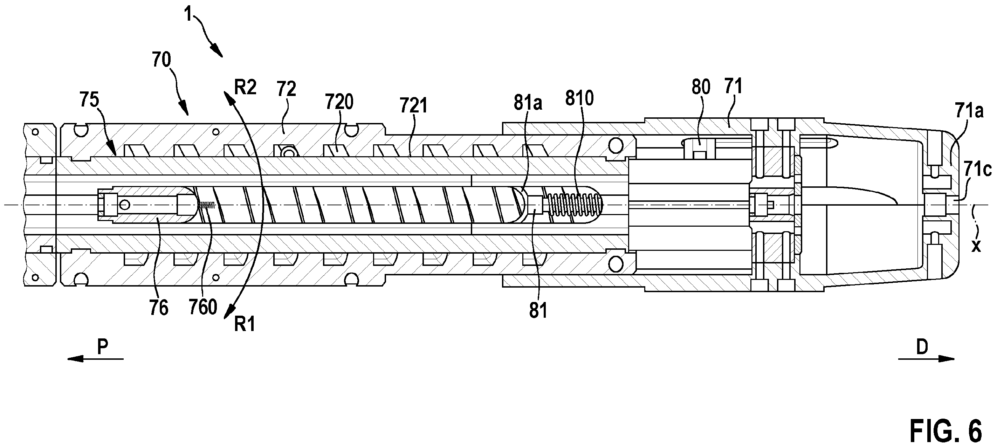

- Fig. 6: shows a cross-sectional view of a handle of an embodiment of a catheter device according to the present invention comprising a locking element for limiting or allowing a further movement of the traveler of the handle in the distal direction, wherein the traveler is in a proximal position;

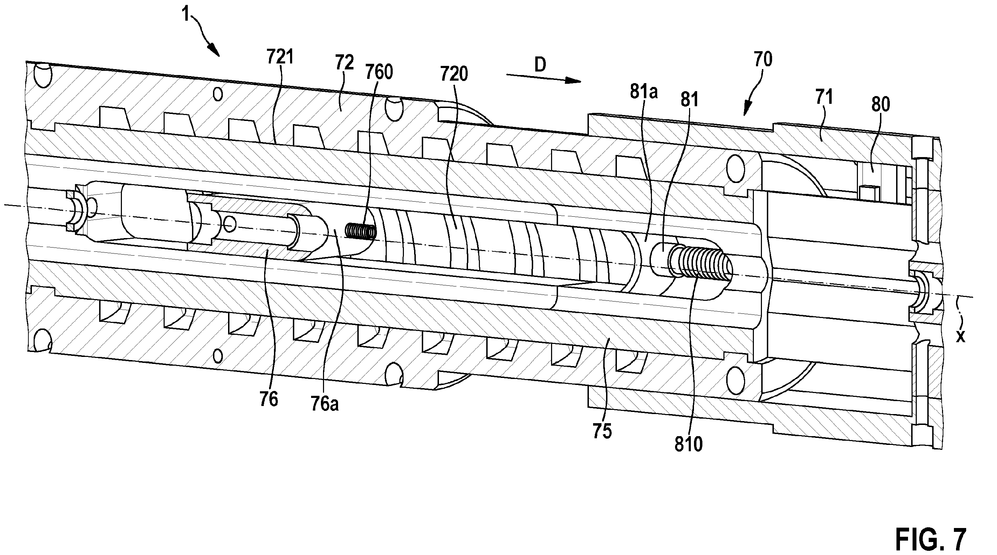

- Fig. 7: shows a perspective cross-sectional view of the handle shown in Fig. 6, wherein the traveler and thus the capsule are not fully retracted;

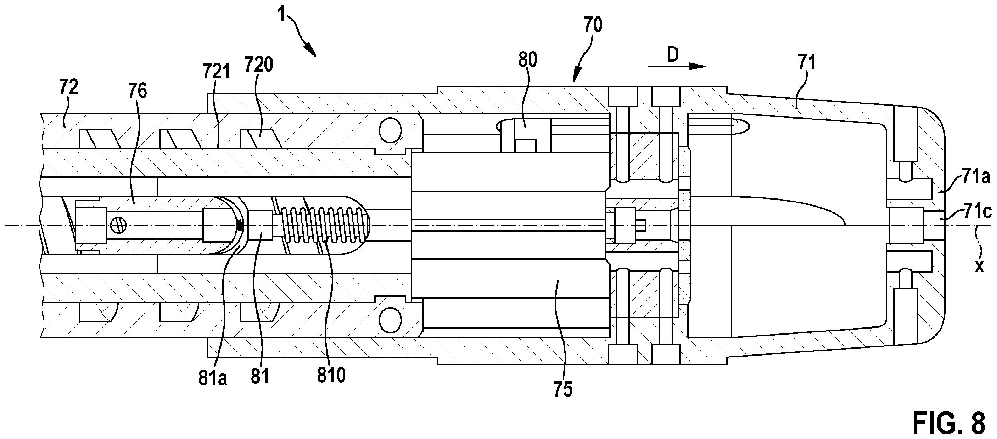

- Fig. 8: shows a cross-sectional view of the handle shown in Figs. 6 and 7, wherein further movement of the traveler is limited by the locking element; in case of non-deformed outer and inner sheaths this position of the capsule corresponds to the closed state of the capsule where the implant is fully covered and can be moved to the implantation site using the catheter device;

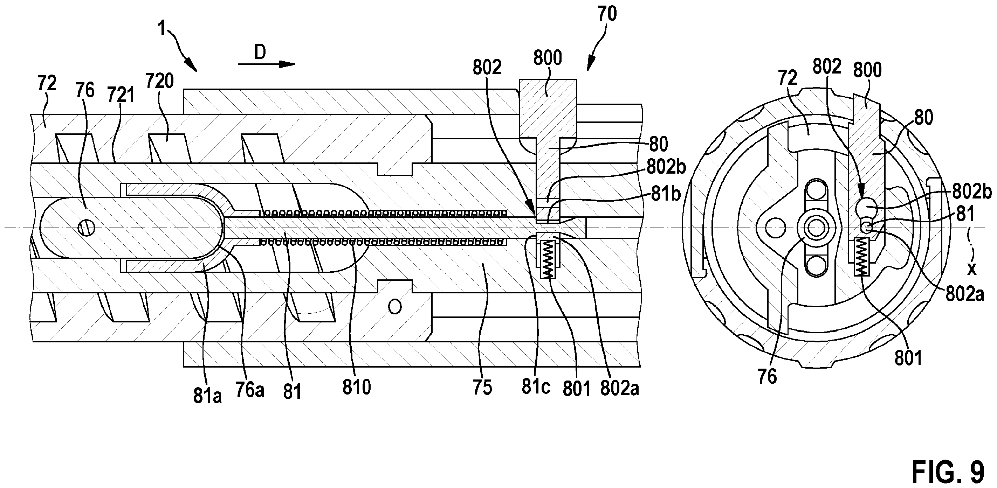

- Fig. 9: shows further views of the state of the handle of the catheter device shown in Fig. 8;

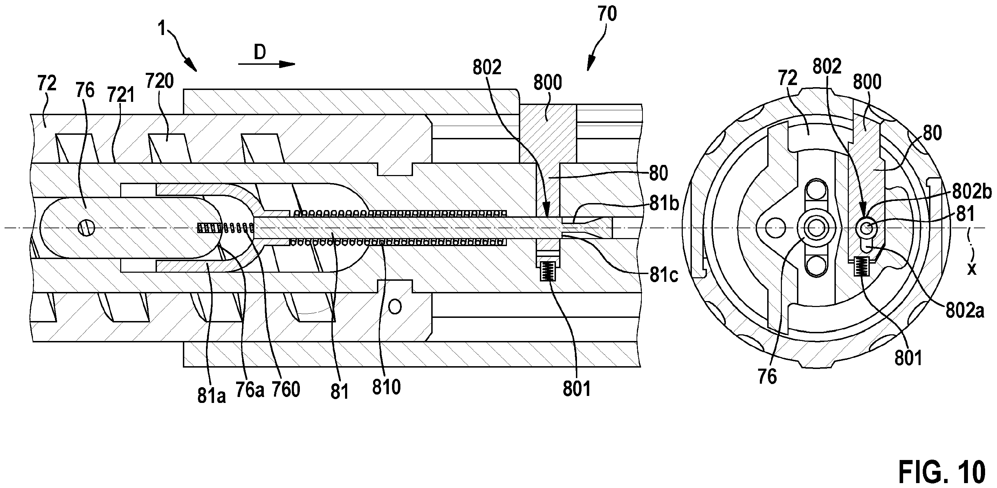

- Fig. 10: shows the situation where - compared to Figs. 8 and 9 - the locking element has been brought from the first state to the second state such that the locking element releases the rod and the pretensioned first spring element pushes the rod further into the opening of the locking element which keeps the locking element in the second state and allows further movement of the traveler and capsule in the distal direction;

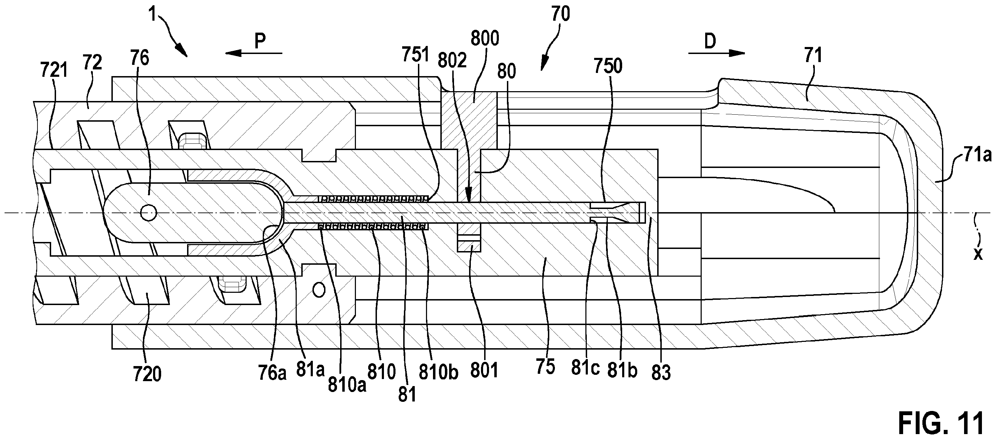

- Fig. 11: shows a cross sectional view of the handle of the catheter device shown in Figs. 6 to 10, wherein the further movement of the traveler in the distal direction is stopped by the rod that butts against a stop of the handle (i.e. maximal overtravel of the capsule has been reached); and

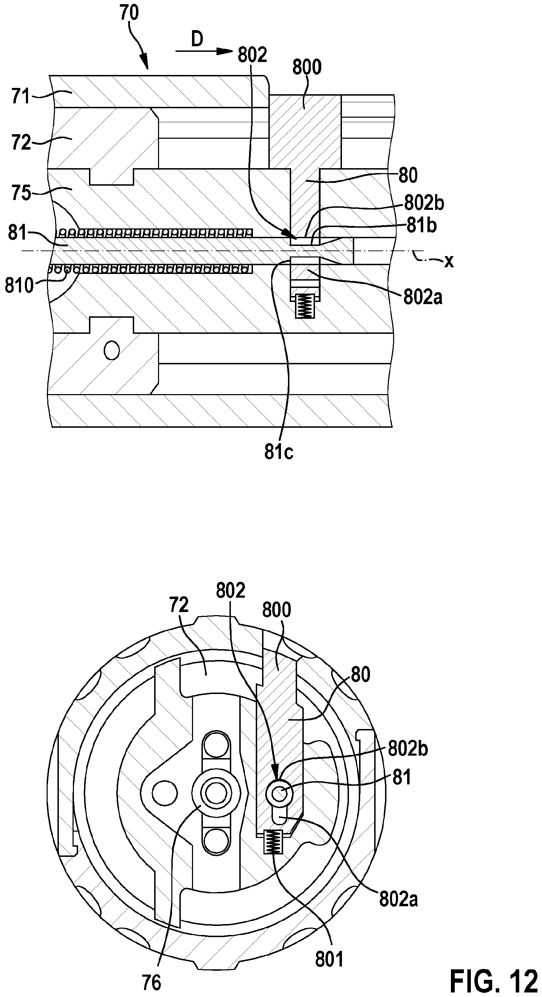

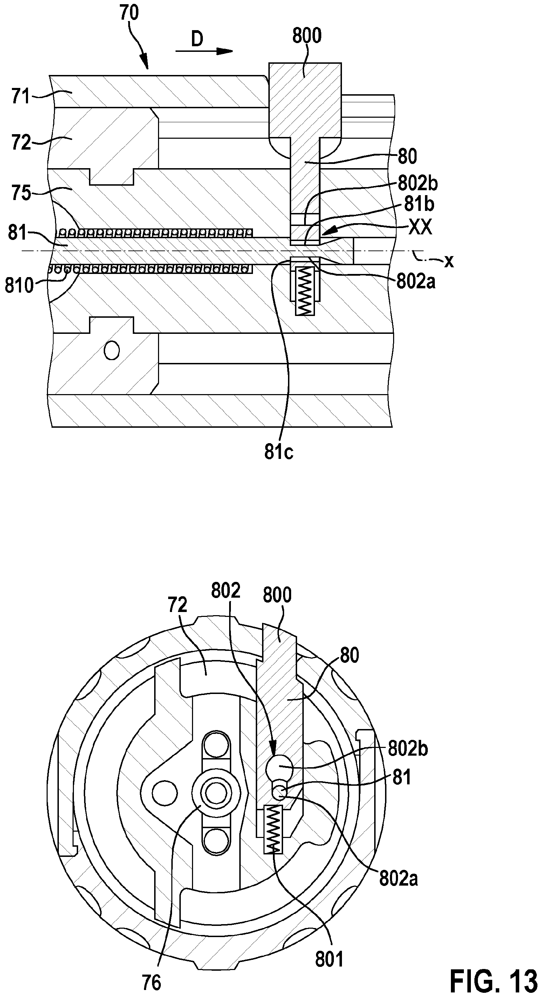

- Figs. 12 to 13: show the return of the locking element from the second state to the first state by moving the traveler in the proximal direction, which due to the action of the second spring element cause a narrow section of the rod to be again positioned in a narrow portion of an opening of the locking element so that the locking element can be moved into its first position by means of the pretensioned third spring element.

The present invention enables further travel of a capsule 40 of a catheter device 1 so that the capsule 40 can properly resheath a partially deployed implant 100 even in case an outer and inner sheath 10, 20 of the catheter device 1 have been permanently deformed due to axial loads during the implantation procedure.

The principle mechanism of the invention is illustrated in Figs. 3 to 5 and will be explained below in context with functions of the handle 70 and other components of the catheter device 1.

Particularly, Fig. 1 shows a distal portion of an embodiment of a catheter device 1 according to the present invention, which comprises a handle 70 that can be used to control functions of the catheter device 1. The handle is e.g. illustrated in Figs. 6 to 13 and will be described in more detail further below. The catheter device 1 is configured to position a medical implant 100, particularly a prosthetic heart valve prosthesis such as a prosthetic aortic heart valve at an implantation site (e.g. anulus of a native aortic valve that is to be replaced). As indicated in Fig. 2, the implant 100 can comprise a self-expanding stent 101 with a tissue-based valve that can comprise three valve leaflets connected to the stent 101. Fig. 2 shows the stent 101 in a crimped state, wherein the leaflets (not shown in Fig. 2) can be made out of a biological tissue. The stent 101 preferably comprises a plurality of interconnected struts 103, so that the stent 101 forms a circumferential scaffold comprising a plurality of lateral openings. The catheter device 1 can facilitate prosthesis delivery as well as axial positioning and angular orienting. Furthermore, the handle 70 can enable partial deployment, full deployment and recapturing (resheathing) of the medical implant / prosthetic heart valve 100.

Particularly, the handle 70 can be adapted to manipulate, particularly steer, four sheaths 20, 10, 60, and 90 as shown in Fig. 2, namely: an inner sheath 20 (also denoted as inner shaft 20) enclosing a guidewire lumen 21, a deflection sheath 60 (also denoted as deflection shaft 60) enclosing the inner sheath 20, an outer sheath 10 (also denoted as outer shaft 10) enclosing the inner sheath 20 and the deflection sheath 60, as well as a stabilizing sheath 90 (also denoted as stabilizing shaft or short "stabilizer" 90) enclosing a proximal section of the inner sheath 20, deflection sheath 60 and outer sheath 10. Particularly, each sheath 10, 20, 60 forms a tubular member.

As indicated in Fig. 2, the deflection sheath 60 can be deflected with a pulling member such as a pull wire 62 (for example a stainless-steel wire) that can be connected to a distal end section 60a of the deflection sheath 60. The pull wire 62 is arranged in a lumen of the deflection sheath 60. The deflection sheath 60 can comprise lateral openings so that the deflection sheath can be deflected by tensioning the pull wire 62. Particularly, in this way, the deflection sheath 60 can be deflected beyond 180°. In the letter case, the deflection sheath comprises a u-shaped distal end section 60a.

The inner and the outer sheath 20, 10 can be moved relative to the deflection sheath 60 (and relative to the stabilizing sheath 90). Additionally, the outer sheath 10 can be moved relative to the inner sheath 20. The stabilizing sheath 90 is stationary and fixed to a grip portion 71 of the handle 70 (cf. Figs. 6 to 13). The stabilizing sheath 90 does only bridge the movements of the other sheaths 10, 20, 60 to the anatomy of the patient and/or an introducer, if necessary. Particularly, the deflection sheath 60 is shorter than the inner and the outer sheath 20, 10. Further, a capsule 40 can be connected to a distal end 10a of the outer sheath 10 (e.g. via a capsule connector 52, cf. Fig. 2), wherein the capsule 40 can be larger in diameter (perpendicular to the longitudinal axis x along which the sheaths 10, 20, 60, 90 extend) than a proximal section of the outer sheath 10.

For delivery using the catheter device 1, the medical implant 100, here an aortic prosthetic heart valve 100, is placed on a support element 30 that is connected to a distal end section 20a of the inner sheath 20 and covered by the capsule 40. The support element 30 can be connected to the inner sheath 20 via a connector 50 to which the heart valve prosthesis 100 is releasably connectable for delivery to an implantation site when it is arranged in the capsule 40. The guidewire lumen 21 can be formed by a tubing 22 that protrudes out of the inner sheath 20 at the distal end 20a of the inner sheath 20, extends through the connector 50 and support element 30, and connects to a catheter tip 24 to which the tubing 22 is connected. Furthermore, the catheter tip 24 can comprise an opening 25 formed in a distal end 24a of the catheter tip 24, so that the guidewire can exit the guidewire lumen 21 via said opening 25 (cf. Figs. 1 and 2).

The movements and functions of the catheter device 1 can be realized with the handle 70 of the catheter device 1 (cf. Figs. 6 to 13), to this end, the handle 70 can comprise:
- a stationary grip portion 71 configured for manually holding the handle 70 and to move the entire catheter device 1;
- a rotatable deployment knob 72 for deploying and releasing the implant 100 by moving the outer sheath 10 in a proximal direction P with respect to the inner sheath 20 and the implant 100.

The handle 70 can further comprise actuating means for achieving a simultaneous movement of the inner and outer sheath 20, 10 as well as for deflecting the sheaths 10, 20 using the deflection sheath 60 and pull wire 62.

Preferably, the knob 72 is rotatable about the longitudinal axis x of the catheter device 1, along which axis x the handle 70 extends.

Furthermore, the grip portion 71 comprises an opening 71c at a distal end 71a of the grip portion 71 through which all sheaths 10, 20, 60, 90 extend into the handle 70 at the distal end 71a of the grip portion 71 (the sheaths are not shown in Figs. 6 to 13).

For deflecting the deflection sheath 60, an actuating means such as a deflection knob can be provided on the handle 70 that can be operatively connected to a distal end section 60a of the deflection sheath 60 via the pull wire 62, so that the deflection sheath 60 and thereby the inner and the outer sheath 20, 10 are deflected, i.e. bent, to adjust an angular orientation of the medical implant 100 when the deflection knob is rotated about the longitudinal axis x which causes tensioning or loosening of the pull wire 62 depending on the direction of the rotation.

Further, for moving the inner and the outer sheath 20, 10 simultaneously, the handle 70 can comprise a guiding member (also denoted as handle core) 75 (cf. Figs. 6 to 13) that is movable with respect to the grip portion 71 along the longitudinal axis x, wherein the inner and the outer sheath 20, 10 are connected to the guiding member 75, and an actuating means such as an axial positioning knob can be operatively connected to the guiding member 75 such that the inner and the outer sheath 20, 10 are simultaneously moved with the guiding member 75 with respect to the grip portion 71 (and with respect to the deflection sheath 60 and stabilizing sheath 90) along the longitudinal axis x when the axial positioning knob is rotated about the longitudinal axis x.

Further, the handle 70 comprises a traveler 76 (also denoted as outer sheath hub), wherein the outer sheath 10 is connected to the traveler 76, and wherein the deployment knob 72 is operatively connected to the traveler 76 such that the traveler 76 and thereby the outer sheath 10 are moved along the longitudinal axis x with respect to the inner sheath 20, and so as to deploy the medical implant 100 when the deployment knob 72 is rotated in a first rotation direction of the deployment knob 72 (cf. Figs 6 to 13).

If the physician is satisfied with the positioning of the catheter device 1, deployment of the prosthesis 100 is started by the physician. In this regard, an axial movement of the outer sheath 10 (and therewith of the capsule 40 and implant 100) in the proximal direction P relative to all other sheaths 20, 60, 90 and the grip portion 71 of handle 70 releases the implant 100; e.g. a prosthetic aortic heart valve. This movement of the outer sheath 10 can be controlled by means of the deployment knob 72 (cf. Figs. 6 to 13), wherein rotation of the deployment knob 72 moves the traveler 76 to which the outer sheath 10 is connected. The movement of the outer sheath 10 and therewith of the capsule 40 in the proximal direction P releases the implant 100.

For releasing the implant 100 based on the above-described movement of the outer sheath 10 and capsule 40, the connector 50 connected to a distal end 20a of the inner sheath 20 can comprise a recess 51, wherein the at least one fastening element 102 of the medical implant 100 is engaged with the at least one recess 51 as long as the capsule 40 covers the at least one recess 51 of the connector 50 and the at least one fastening element 102 that engages with the at least one recess 51 when the prosthetic heart valve 100 is arranged on the support 30 (cf. Fig. 2). The at least one fastening element 102 can be formed by a portion of the self-expandable stent 101 of the prosthetic heart valve 100. Particularly, as shown in Fig. 2, the at least one fastening element 102 can be connected to at least one strut 103 of the stent 101 at a proximal end of the stent 101. For example, the stent 101 can comprise three such fastening elements 102. In this case, the connector 50 comprises three corresponding recesses 51.

Once the capsule 40 is completely removed from the implant 100 and does no longer cover the at least one fastening element 102 and the corresponding recess 51 of the connector 50 (cf. lower part of Fig. 8) the at least one fastening element 102 disengages with the connector 50 due to the self-expanding property of the implant 100 / stent 101, which releases the implant 100 at the implantation site. After complete deployment and release of the implant 100 from the catheter device 1, resheathing of the implant 100 is no longer possible.

In case the implant 100 is only partially deployed and still connected to the connector 50, resheathing of the implant 100 is possible. However, due to high axial loads acting on the inner and outer sheath 20, 10 during the implantation procedure, the sheaths 20, 10 can become permanently deformed in the direction of the longitudinal axis x during operation of the catheter device 1. This usually corresponds to a compression of the outer sheath 10 and an elongation of the inner sheath 10; which means that it is no longer possible to properly close the capsule 40, i.e., to completely cover the implant 100 with the capsule 40. Typically, a gap can result between the tip 24 and a distal edge of the capsule 40 which means that the resheathing cannot be properly completed.

In order to prevent this outcome, the present invention provides the capsule 40 with an additional travel which is denoted as overtravel and corresponds to a further movement of the capsule 40 in the distal direction D past the initial limit of the capsule 40.

As shown in Figs. 3 to 5, in order to achieve this additional travel, the movement of the outer sheath 10 and therewith of the capsule 40 is coupled via e.g. the traveler 76 to an elongated rod 81 of the handle 70.

With the axial movement in the distal direction D during closing of the capsule 40, the rod 80 is reaching a rigid barrier in form of a locking element 80 which is inhibiting further axial travel of the rod 81 and therewith of the traveler 76 in the distal direction D. A first spring element 706 between the traveler 76 and the rod 81 is loaded in this position (cf. Fig. 3).

The locking element 80 can be configured to allow passage of the rod 81 through an opening 802 and can be configured to comprise a push button 800 for moving the locking element 80 perpendicular to the longitudinal axis x/ distal direction D. The pushing of the locking element 80 loads the third spring element 801. Once the barrier 80 is removed, the rod 81 is pushed forward by the pretensioned first spring element 760. According to an embodiment of the present invention, the unloading of the first spring element 760 is audible and the change in position of the push button 800 is visible (cf. Fig 4).

Subsequently, the user can move the traveler 76 and therewith the outer sheath 10 and capsule 40 forward in the distal direction D and the rod 81 moves further through the opening 802 of the locking element/barrier 80. Using this overtravel, the user carefully closes the capsule 40 (capsule 40 properly aligned with the atraumatic tip 24 in a gapless fashion) and thereby pretensions the first spring element 760 as well as the second spring element 810 (cf. Fig. 5).

In the next step, in case the user is satisfied with the repositioned implant, the user is releasing the implant 100 again. The outer sheath 10 is moved in the opposite direction (proximal direction P) and the implant 100 is deployed. After this second deployment it is possible that the user needs to resheath the prosthesis again. Therefore, the already used locking mechanism must be re-activated.

This lock reactivation is achieved by means of the second and third spring element 810, 801. Starting from Fig. 5, the traveler 76 that connects to the outer sheath 10 and capsule 40 is now being moved in the proximal direction P (i.e. to the left). This allows the second spring element 810 to force the rod 81 to follow the traveler 76. Once the rod 81 has traveled past the locking element / barrier 80, the third spring element 801 pushes the locking element / barrier 80 back into the locked position (first state). According to an embodiment, the relaxation of the third spring element 801 is audible and the position change of the locking element / barrier 80 is visible via the push button 800. With this mechanism, the lock/unlock cycle can be repeated.

This mechanism can be integrated in various ways into a handle 70 of a catheter device 1. In the following an embodiment of such an integration is described with reference to Figs. 6 to 13.

As shown in Fig. 6 in conjunction with Figs. 1 and 2, the catheter device 1 comprises an outer sheath 10 extending along a longitudinal axis x of the catheter device 1 and surrounding a lumen 11 of the outer sheath 10, an inner sheath 20 extending along the longitudinal axis x, wherein the inner sheath 20 is arranged in the lumen 11 of the outer sheath 10 and connected to a support element 30 for supporting the medical implant 100, a capsule 40 connected to a distal end 10a of the outer sheath 10 for covering the medical implant 100 when the medical implant 100 is arranged on the support element 30, a handle 70 comprising: a grip portion 71 for manually holding the handle 70, a rotatable deployment knob 72, and a traveler 76, wherein the outer sheath 10 (cf. Fig. 2, not shown in Figs. 6 to 13) is connected to the traveler 76, wherein the deployment knob 72 comprises a helical groove 720 formed in an inside 721 of the deployment knob 72, wherein the traveler 76 engages into the helical groove 720 of the deployment knob 72 such that the traveler 76 and thereby the outer sheath 10 are moved in a proximal direction P along the longitudinal axis x with respect to the inner sheath 20 when the deployment knob 72 is rotated in a first rotation direction R1, so that the capsule 40 is pulled away from the medical implant 100 in the proximal direction P to deploy the medical implant 100, and such that the traveler 76 and thereby the outer sheath 10 are moved in the distal direction D along the longitudinal axis x with respect to the inner sheath 20 when the deployment knob 72 is rotated in an opposite second rotation direction R2 (cf. Fig. 6), so that the capsule 40 is moved over the medical implant 100 to cover the medical implant 100. Furthermore, the handle 70 comprises a locking element 80 that is configured to be moved from a first to a second state, wherein the locking element 80 limits a movement of the traveler 76 and therewith of the capsule 40 in the distal direction D in the first state, and wherein the locking element 80 allows further movement of the traveler 76 and therewith of the capsule 40 in the distal direction D in the second state. Particularly, the inner sheath (cf. Fig 2, not shown in Figs. 6 to 13) is stationary during the deploy/resheath cycle which can include an unlock/lock cycle.

As stated above, the deploy traveler 76 is connected to the outer sheath 10 and is moved in axial direction x by the rotation of the deployment knob 72. On the front side 76a of the traveler 76, a first spring element 706 is connected to the traveler 76 that protrudes from the front side 76a in the distal direction D and moves with the traveler 76 (cf. Fig. 7).

Particularly, the traveler 76 moves in the distal direction D (to the right) until it reaches an elongated slidable rod 81 that is also denoted as overtravel traveler. The state depicted in Figures 8 and Figure 9 essentially corresponds to the state shown in Fig. 3.

When the traveler 76 has reached the rod 81 as shown in Fig. 8, the front side 76a is particularly enclosed in a form-fitting manner by a concave face side of a proximal end portion 81a of the rod 81. The first spring element 760 is therefore fully loaded, i.e., pretensioned against the rod 81. The second spring element 810 that surrounds the rod 81 is not yet loaded since the rod 81 is stopped by the locking element 80 as shown in Fig. 9.

Fig. 9 shows a side (left) and a front (right) view of the lock mechanism. The rod 81 is pushed in the distal direction D by the traveler 76/first spring element 760 (not visible in Fig. 9). The locking element 80 is blocking the path of the rod 81, wherein a third spring element 801 below the locking element 80 is holding the locking element 80 in the default first state corresponding to the locked state of the locking element 80.

Particularly, the locking element 80 comprises an opening 802 having a narrow portion 802a and an adjacent wider portion 802b. Correspondingly, the rod 81 comprises a distal portion comprising a narrow section 81b having a diameter that is smaller than a diameter of an adjacent proximal portion of the rod 81 so that the rod 81 forms a step 81c (e.g. a circumferential step) at said section 81b, wherein when the locking element 80 is in the first (locked) state said narrow section 81b of the rod 81 is arranged in the narrow portion 802a of the opening 802 and the rod 81 cannot be moved further through the opening 802 since the step 81c butts against the locking element 80, namely against an edge of the narrow portion 802a of the opening 802.

According to Fig. 10, the additional travel of the traveler 76 / capsule 40 is activated by pushing down a top side 800 of the locking element 80 which forms a push button 800. The corresponding downward movement of the locking element 80 arranges the rod 81 in the wider portion 802b of the opening 802 which clears the path for the rod 81 to travel through, since the wider portion 802b comprises a diameter that does not allow blocking of the step 81c of the rod 81.

Now, with the locking element 80 being in the second (open) state, the traveler's 76 first spring element 706 pushes the rod 81 forward through the opening 802 of the locking element 80. Particularly, the user can hear/feel the jump of the rod 81 as feedback for successful unlocking. Due to the button 800/locking element 80 being pushed into the second state, the third spring element 801 is now loaded.

The elongated rod 81 can now travel freely through the wider portion 802b of the opening 802 of the locking element 80. The distal movement of the deploy traveler 76 (overtravel) can be used by rotating the deployment knob 72 further in the first rotation direction R1 until the first spring element 760 and the second spring element 810 are fully compressed or the rod 81 hits a stop 83 provided on the handle 70.

Particularly, the locking element 80 being in the second (unlocked) state allows a specific extra overtravel. According to an embodiment, this overtravel is in the range from 10 mm to 30 mm. Particularly, the overtravel amounts to 20 mm in an embodiment. Fig. 11 shows the maximum overtravel/possible further movement of the capsule 40. In this case the first and the second spring element 760, 810 are fully compressed and the rod 81 reaches the stop 83.

Particularly, as shown in Fig. 11, the rod 81 is guided by a guiding member 75 that is also denoted as handle core 75. The guiding member 75 comprises an elongated hole 750 into which the rod 81 extends and which guides the rod 81 upon its movement long the longitudinal axis x. A bottom 83 of the hole 750 forms the hard stop for the rod 81. The second spring element 810 that surrounds the rod 81 butts with a first end 810a against a circumferential step 751 formed on an internal surface of the hole 750, and with a second end 810b against the proximal end portion 81a of the rod 81. In this way, the pretensioned second spring element 810 can push the rod 81 in the proximal direction P in case the traveler 76 is moved back in the proximal direction P with the locking element 80 being in the second (open) state.

After successful recapturing of the implant 100 with the help of the overtravel of the capsule 40, the user opens the capsule 40 (i.e. moves it proximal) for a second deploy attempt. The deploy traveler 76 then moves in the proximal direction (to the left in Fig. 11) and the second spring element 810 ensures that the entire rod 81 is moving proximal, too. If the traveler 76 reaches the initial position before the overtravel activation, the narrow section 81b of the rod 81 is aligned with the opening 802 of the locking element 80 again. The third spring element 801 can thus push the locking element 80 back up in its original position (first state). This means that the narrow section 81b of the rod shifts to the narrow portion 802a of the opening 802 of the locking element 80 such that the rod 81 presses with its step 81c against the locking member 80 in case an attempt to move the traveler 76 further distal is made. The locking element / mechanism 80 can now be reused.

Particularly, the movement of the locking element 80 is visible as well as audible.

The solution according to the present invention combines increased safety with good usability. Particularly, the locking element can be operated in a stable manner and its states can be detected either visually and/or acoustically.

## Claims

1. A catheter device (1) for implanting a medical implant (100), comprising:
- an outer sheath (10) extending along a longitudinal axis (x) of the catheter device (1) and surrounding a lumen (11) of the outer sheath (10),
- an inner sheath (20) extending along the longitudinal axis (x), wherein the inner sheath (20) is arranged in the lumen (11) of the outer sheath (10) and connected to a support element (30) for supporting the medical implant (100) or to a connector (50),
- a capsule (40) connected to a distal end (10a) of the outer sheath (10) for covering the medical implant (100) when the medical implant (100) is arranged on the support element (30) or the connector (50),
- a handle (70) comprising: a grip portion (71) for manually holding the handle (70), a rotatable deployment knob (72), and a traveler (76), wherein the outer sheath (10) is connected to the traveler (76), wherein the deployment knob (72) comprises a helical groove (720) formed in an inside (721) of the deployment knob (72), wherein the traveler (76) engages into the helical groove (720) of the deployment knob (72) such that the traveler (76) and thereby the outer sheath (10) are moved in a proximal direction (P) along the longitudinal axis (x) with respect to the inner sheath (20) when the deployment knob (72) is rotated in a first rotation direction (R1), so that the capsule (40) is pulled away from the medical implant (100) in the proximal direction (P) to deploy the medical implant (100), and such that the traveler (76) and thereby the outer sheath (10) are moved in the distal direction (D) along the longitudinal axis (x) with respect to the inner sheath (20) when the deployment knob (72) is rotated in an opposite second rotation direction (R2), so that the capsule (40) is moved over the medical implant (100) to cover the medical implant (100), wherein the handle (70) comprises a locking element (80) that is configured to be moved from a first to a second state, wherein the locking element (80) limits a movement of the traveler (76) and therewith of the capsule (40) in the distal direction (D) in the first state, and wherein the locking element (80) allows a further movement of the traveler (76) and therewith of the capsule (40) in the distal direction (D) in the second state.

2. The catheter device according to claim 1, wherein in the first state the locking element (80) is configured to set a limit to the movement of the traveler (76) in the distal direction (D) such that the capsule (40) completely covers the implant (100) when the traveler is positioned at said limit and the inner and the outer sheath (20, 10) have not yet been permanently deformed during operation of the catheter device (1) and each comprise their initial length in the direction of the longitudinal axis (x).

3. The catheter device according to claim 1 or 2, wherein in the second state the locking element (80) is configured to allow further movement of the traveler (76) and therewith of the capsule (40) in the distal direction (D) such that the implant is completely coverable by the capsule (40) despite a permanent compression of the outer sheath (10) and/or despite a permanent elongation of the inner sheath (20) in the direction of the longitudinal axis (x).

4. The catheter device according to one of the preceding claims, wherein the handle (70) comprises an elongated rod (81) that is slidable along the longitudinal axis (x), wherein when the locking element (80) is in its first state, the rod (81) is configured to be pressed by the traveler (76) against the locking element (80) to limit the movement of the traveler (76) in the distal direction (D), wherein a first spring element (760) connected to the traveler (76) is arranged between the traveler (76) and the rod (81) and is pretensioned against the rod (81) when the traveler (76) presses against the locking element (80).

5. The catheter device according to claim 4, wherein the locking element (80) is configured to release the rod (81) when the locking element (80) is moved from the first state to the second state, wherein the rod (81) is pushed in the distal direction (D) by the first spring element (760).

6. The catheter device according to claim 4 or 5, wherein when the locking element (80) is in the second state, the further movement of the traveler (76) in the distal direction (D) pushes the rod (81) against a stop (83) to finally limit the further movement of the traveler (76) and therewith of the capsule (40) in the distal direction (D), wherein the first spring element (760) is pretensioned by the traveler (76) against the rod (81) and a second spring element (810) is pretensioned by the rod (81) against a guiding member (75) that guides the rod (81) and forms said stop (83).

7. The catheter device according to one of the preceding claims, wherein the handle (70) comprises an actuating element (800) configured to be manually actuated by a user, wherein the actuating element (800) is operatively connected to the locking element (80) such that the locking element (80) is moved from the first state to the second state when the actuating element (800) is actuated by a user.

8. The catheter device according to one of the preceding claims, wherein the handle (70) comprises a third spring element (801), wherein the third spring element (801) is configured to be pretensioned against the locking element (80) when the latter is moved from the first state to the second state.

9. The catheter device according to claim 4 or according to one of the claims 5 to 8 if referring to claim 4, wherein the locking element (80) comprises an opening (802) having a narrow portion (802a) and an adjacent wider portion (802b), and wherein the rod (81) comprises a distal portion comprising a narrow section (81b) having a diameter that is smaller than a diameter of an adjacent proximal portion of the rod (81) so that the rod (81) forms a step (81c), wherein when the locking element (80) is in the first state said narrow section (81b) of the rod (81) is arranged in the narrow portion (802a) of the opening (802) and the rod (81) is configured to be pressed by the traveler (76) against the locking element (80) with said step (81c) to limit the movement of the traveler (76) in the distal direction (D).

10. The catheter device according to claims 4 and 9, wherein when the locking element (80) is moved from the first state to the second state, the rod (81) is arranged in the wide portion (802b) of the opening (802) so that the step (81c) is allowed to move through the wide portion (802b) of the opening (802) of the rod (81) by the action of the first spring element (760).

11. The catheter device according to claims 4, 6, and 8, and according to claim 9 or 10, wherein, when the second spring element (810) is pretensioned, moving the traveler (76) and therewith the capsule (40) in the proximal direction (P) causes the second spring element (810) to move the rod (81) in the proximal direction (P), too, such that said narrow section (81b) of the rod (81) moves into the wide portion (802b) of the opening (802) of the locking element (80) at a certain position of the traveler (76) such that the third spring element (801) moves the locking element (80) back into the first state in which said narrow section (81b) of the rod (81) is arranged in the narrow portion (802a) of the opening (802) of the locking element (80).

12. The catheter device according to claim 6 or according to one of the claims 7 to 11 if referring to claim 6, wherein the deployment knob (72) is rotatably supported on the guiding member (75) of the handle (70).

## Patentansprüche

1. Kathetervorrichtung (1) zum Implantieren eines medizinischen Implantats (100), umfassend:
- eine Außenhülle (10), die sich entlang einer Längsachse (x) der Kathetervorrichtung (1) erstreckt und ein Lumen (11) der Außenhülle (10) umgibt,
- eine sich entlang der Längsachse (x) erstreckende Innenhülle (20), wobei die Innenhülle (20) im Lumen (11) der Außenhülle (10) angeordnet und mit einem Stützelement (30) zur Abstützung des medizinischen Implantats (100) oder mit einem Verbinder (50) verbunden ist,
- eine Kapsel (40), die mit einem distalen Ende (10a) der äußeren Hülle (10) verbunden ist, um das medizinische Implantat (100) zu bedecken, wenn das medizinische Implantat (100) auf dem Trägerelement (30) oder dem Verbinder (50) angeordnet ist,
- einen Griff (70), umfassend: einen Griffabschnitt (71) zum manuellen Halten des Griffs (70), einen drehbaren Entfaltungsknopf (72) und einen Läufer (76), wobei die Außenhülle (10) mit dem Läufer (76) verbunden ist, wobei der Entfaltungsknopf (72) eine schraubenförmige Nut (720) aufweist, die in einer Innenseite (721) des Entfaltungsknopfs (72) ausgebildet ist, wobei der Läufer (76) in die schraubenförmige Nut (720) des Entfaltungsknopfes (72) eingreift, so dass der Läufer (76) und dadurch die äußere Hülle (10) in einer proximalen Richtung (P) entlang der Längsachse (x) in Bezug auf die innere Hülle (20) bewegt werden, wenn der Entfaltungsknopf (72) in einer ersten Drehrichtung (R1) gedreht wird, so dass die Kapsel (40) von dem medizinischen Implantat (100) in der proximalen Richtung (P) weggezogen wird, um das medizinische Implantat (100) zu entfalten, und so, dass der Läufer (76) und damit die äußere Hülle (10) in der distalen Richtung (D) entlang der Längsachse (x) in Bezug auf die innere Hülle (20) bewegt werden, wenn der Entfaltungsknopf (72) in einer entgegengesetzten zweiten Drehrichtung (R2) gedreht wird, so dass die Kapsel (40) über das medizinische Implantat (100) bewegt wird, um das medizinische Implantat (100) abzudecken, wobei der Griff (70) ein Verriegelungselement (80) umfasst, das so konfiguriert ist, dass es von einem ersten in einen zweiten Zustand bewegt werden kann, wobei das Verriegelungselement (80) eine Bewegung des Läufers (76) und damit der Kapsel (40) in der distalen Richtung (D) in dem ersten Zustand begrenzt, und wobei das Verriegelungselement (80) eine weitere Bewegung des Läufers (76) und damit der Kapsel (40) in der distalen Richtung (D) in dem zweiten Zustand ermöglicht.

2. Kathetervorrichtung nach Anspruch 1, wobei im ersten Zustand das Verriegelungselement (80) so ausgebildet ist, dass es der Bewegung des Läufers (76) in distaler Richtung (D) eine Grenze setzt, so dass die Kapsel (40) das Implantat (100) vollständig abdeckt, wenn der Läufer an dieser Grenze positioniert ist und die Innen- und die Außenhülle (20, 10) im Betrieb der Kathetervorrichtung (1) noch nicht dauerhaft verformt wurden und jeweils ihre Ausgangslänge in Richtung der Längsachse (x) aufweisen.

3. Kathetervorrichtung nach Anspruch 1 oder 2, wobei im zweiten Zustand das Verriegelungselement (80) so ausgestaltet ist, dass es eine weitere Bewegung des Läufers (76) und damit der Kapsel (40) in distaler Richtung (D) zulässt, so dass das Implantat trotz einer dauerhaften Kompression des Außenmantels (10) und/oder trotz einer dauerhaften Dehnung des Innenmantels (20) in Richtung der Längsachse (x) vollständig von der Kapsel (40) abdeckbar ist.

4. Kathetervorrichtung nach einem der vorhergehenden Ansprüche, wobei der Handgriff (70) eine längliche Stange (81) aufweist, die entlang der Längsachse (x) verschiebbar ist, wobei die Stange (81) im ersten Zustand des Verriegelungselements (80) so ausgebildet ist, dass sie durch den Läufer (76) gegen das Verriegelungselement (80) gedrückt wird, um die Bewegung des Läufers (76) in distaler Richtung (D) zu begrenzen, wobei ein erstes Federelement (760), das mit dem Läufer (76) verbunden ist, zwischen dem Läufer (76) und der Stange (81) angeordnet ist und gegen die Stange (81) vorgespannt ist, wenn der Läufer (76) gegen das Verriegelungselement (80) drückt.

5. Kathetervorrichtung nach Anspruch 4, wobei das Verriegelungselement (80) so konfiguriert ist, dass es die Stange (81) freigibt, wenn das Verriegelungselement (80) von dem ersten Zustand in den zweiten Zustand bewegt wird, wobei die Stange (81) durch das erste Federelement (760) in die distale Richtung (D) gedrückt wird.

6. Kathetervorrichtung nach Anspruch 4 oder 5, wobei im zweiten Zustand des Verriegelungselements (80) die weitere Bewegung des Läufers (76) in der distalen Richtung (D) die Stange (81) gegen einen Anschlag (83) drückt, um die weitere Bewegung des Läufers (76) und damit der Kapsel (40) in der distalen Richtung (D) endgültig zu begrenzen, wobei das erste Federelement (760) durch den Läufer (76) gegen die Stange (81) vorgespannt ist und ein zweites Federelement (810) durch die Stange (81) gegen ein Führungselement (75) vorgespannt ist, das die Stange (81) führt und den Anschlag (83) bildet.

7. Kathetervorrichtung nach einem der vorhergehenden Ansprüche, wobei der Handgriff (70) ein Betätigungselement (800) aufweist, das so ausgestaltet ist, dass es von einem Benutzer manuell betätigt werden kann, wobei das Betätigungselement (800) mit dem Verriegelungselement (80) in Wirkverbindung steht, so dass das Verriegelungselement (80) von dem ersten Zustand in den zweiten Zustand bewegt wird, wenn das Betätigungselement (800) von einem Benutzer betätigt wird.

8. Kathetervorrichtung nach einem der vorhergehenden Ansprüche, wobei der Handgriff (70) ein drittes Federelement (801) aufweist, wobei das dritte Federelement (801) so ausgebildet ist, dass es gegen das Verriegelungselement (80) vorgespannt wird, wenn dieses vom ersten Zustand in den zweiten Zustand bewegt wird.

9. Kathetervorrichtung nach Anspruch 4 oder nach einem der Ansprüche 5 bis 8, wenn auf Anspruch 4 Bezug genommen wird, wobei das Verriegelungselement (80) eine Öffnung (802) mit einem schmalen Abschnitt (802a) und einem angrenzenden breiteren Abschnitt (802b) umfasst, und wobei die Stange (81) einen distalen Abschnitt umfasst, der einen schmalen Abschnitt (81b) mit einem Durchmesser umfasst, der kleiner als ein Durchmesser eines angrenzenden proximalen Abschnitts der Stange (81) ist, so dass die Stange (81) eine Stufe (81c) bildet, wobei, wenn sich das Verriegelungselement (80) im ersten Zustand befindet, der schmale Abschnitt (81b) der Stange (81) in dem schmalen Abschnitt (802a) der Öffnung (802) angeordnet ist und die Stange (81) so konfiguriert ist, dass sie von dem Läufer (76) gegen das Verriegelungselement (80) mit der Stufe (81c) gedrückt wird, um die Bewegung des Läufers (76) in der distalen Richtung (D) zu begrenzen.

10. Kathetervorrichtung nach einem der Ansprüche 4 und 9, wobei, wenn das Verriegelungselement (80) vom ersten Zustand in den zweiten Zustand bewegt wird, die Stange (81) im weiten Abschnitt (802b) der Öffnung (802) angeordnet ist, so dass die Stufe (81c) durch die Wirkung des ersten Federelements (760) durch den weiten Abschnitt (802b) der Öffnung (802) der Stange (81) bewegt werden kann.

11. Kathetervorrichtung nach Anspruch 4, 6 und 8 sowie nach Anspruch 9 oder 10, wobei bei vorgespanntem zweitem Federelement (810) eine Bewegung des Läufers (76) und damit der Kapsel (40) in proximaler Richtung (P) bewirkt, dass das zweite Federelement (810) auch die Stange (81) in proximaler Richtung (P) bewegt, so dass der schmale Abschnitt (81b) der Stange (81) sich in den breiten Abschnitt (802b) der Öffnung (802) des Verriegelungselements (80) bei einer bestimmten Position des Läufers (76) bewegt, so dass das dritte Federelement (801) das Verriegelungselement (80) zurück in den ersten Zustand bewegt, in dem der schmale Abschnitt (81b) der Stange (81) in dem schmalen Abschnitt (802a) der Öffnung (802) des Verriegelungselements (80) angeordnet ist.

12. Kathetervorrichtung nach Anspruch 6 oder nach einem der Ansprüche 7 bis 11, wenn auf Anspruch 6 Bezug genommen wird, wobei der Entfaltungsknopf (72) drehbar an dem Führungselement (75) des Griffs (70) gelagert ist.

## Revendications

1. Dispositif de cathéter (1) pour l'implantation d'un implant médical (100), comprenant:
- une gaine extérieure (10) s'étendant le long d'un axe longitudinal (x) du dispositif de cathéter (1) et entourant une lumière (11) de la gaine extérieure (10),
- une gaine intérieure (20) s'étendant le long de l'axe longitudinal (x), dans laquelle la gaine intérieure (20) est disposée dans la lumière (11) de la gaine extérieure (10) et reliée à un élément de support (30) pour soutenir l'implant médical (100) ou à un connecteur (50),
- une capsule (40) reliée à une extrémité distale (10a) de la gaine extérieure (10) pour recouvrir l'implant médical (100) lorsque l'implant médical (100) est disposé sur l'élément de support (30) ou le connecteur (50),
- une poignée (70) comprenant : une partie de préhension (71) pour tenir manuellement la poignée (70), un bouton de déploiement rotatif (72), et un dispositif de déplacement (76), dans lequel la gaine extérieure (10) est reliée au dispositif de déplacement (76), dans lequel le bouton de déploiement (72) comprend une rainure hélicoïdale (720) formée à l'intérieur (721) du bouton de déploiement (72), dans lequel le dispositif de déplacement (76) s'engage dans la rainure hélicoïdale (720) de la molette de déploiement (72) de sorte que le dispositif de déplacement (76) et donc la gaine extérieure (10) sont déplacés dans une direction proximale (P) le long de l'axe longitudinal (x) par rapport à la gaine intérieure (20) lorsque la molette de déploiement (72) est tournée dans un premier sens de rotation (R1), de sorte que la capsule (40) est éloignée de l'implant médical (100) dans la direction proximale (P) pour déployer l'implant médical (100), et de telle sorte que le dispositif de déplacement (76) et donc la gaine extérieure (10) soient déplacés dans la direction distale (D) le long de l'axe longitudinal (x) par rapport à la gaine intérieure (20) lorsque le bouton de déploiement (72) est tourné dans un deuxième sens de rotation opposé (R2), de telle sorte que la capsule (40) soit déplacée sur l'implant médical (100) pour recouvrir l'implant médical (100), dans lequel la poignée (70) comprend un élément de verrouillage (80) configuré pour être déplacé d'un premier à un second état, dans lequel l'élément de verrouillage (80) limite un mouvement du dispositif de déplacement (76) et de la capsule (40) dans la direction distale (D) dans le premier état, et dans lequel l'élément de verrouillage (80) permet un mouvement supplémentaire du dispositif de déplacement (76) et de la capsule (40) dans la direction distale (D) dans le second état.

2. Dispositif de cathéter selon la revendication 1, dans lequel, dans le premier état, l'élément de verrouillage (80) est configuré pour fixer une limite au mouvement du dispositif de déplacement (76) dans la direction distale (D) de sorte que la capsule (40) recouvre complètement l'implant (100) lorsque le dispositif de déplacement est positionné à ladite limite et que la gaine intérieure et la gaine extérieure (20, 10) n'ont pas encore été déformées de façon permanente pendant le fonctionnement du dispositif de cathéter (1) et comprennent chacune leur longueur initiale dans la direction de l'axe longitudinal (x).

3. Dispositif de cathéter selon la revendication 1 ou 2, dans lequel, dans le second état, l'élément de verrouillage (80) est configuré pour permettre un mouvement supplémentaire du dispositif de déplacement (76) et donc de la capsule (40) dans la direction distale (D), de sorte que l'implant puisse être entièrement recouvert par la capsule (40) malgré une compression permanente de la gaine extérieure (10) et/ou malgré un allongement permanent de la gaine intérieure (20) dans la direction de l'axe longitudinal (x).

4. Dispositif de cathéter selon l'une des revendications précédentes, dans lequel la poignée (70) comprend une tige allongée (81) qui est coulissante le long de l'axe longitudinal (x), dans lequel lorsque l'élément de verrouillage (80) est dans son premier état, la tige (81) est configurée pour être pressée par le dispositif de déplacement (76) contre l'élément de verrouillage (80) afin de limiter le mouvement du dispositif de déplacement (76) dans la direction distale (D), dans lequel un premier élément ressort (760) relié au dispositif de déplacement (76) est disposé entre le dispositif de déplacement (76) et la tige (81) et est précontraint contre la tige (81) lorsque le dispositif de déplacement (76) exerce une pression contre l'élément de verrouillage (80).

5. Dispositif de cathéter selon la revendication 4, dans lequel l'élément de verrouillage (80) est configuré pour libérer la tige (81) lorsque l'élément de verrouillage (80) est déplacé du premier état au second état, la tige (81) étant poussée dans la direction distale (D) par le premier élément de ressort (760).

6. Le dispositif de cathéter selon la revendication 4 ou 5, dans lequel, lorsque l'élément de verrouillage (80) est dans le second état, le mouvement supplémentaire du dispositif de déplacement (76) dans la direction distale (D) pousse la tige (81) contre une butée (83) pour finalement limiter le mouvement supplémentaire du dispositif de déplacement (76) et donc de la capsule (40) dans la direction distale (D), dans lequel le premier élément ressort (760) est précontraint par le dispositif de déplacement (76) contre la tige (81) et un second élément ressort (810) est précontraint par la tige (81) contre un élément de guidage (75) qui guide la tige (81) et forme ladite butée (83).

7. Le dispositif de cathéter selon l'une des revendications précédentes, dans lequel la poignée (70) comprend un élément d'actionnement (800) configuré pour être actionné manuellement par un utilisateur, dans lequel l'élément d'actionnement (800) est relié de manière opérationnelle à l'élément de verrouillage (80) de sorte que l'élément de verrouillage (80) est déplacé du premier état au second état lorsque l'élément d'actionnement (800) est actionné par un utilisateur.

8. Dispositif de cathéter selon l'une des revendications précédentes, dans lequel la poignée (70) comprend un troisième élément de ressort (801), dans lequel le troisième élément de ressort (801) est configuré pour être précontraint contre l'élément de verrouillage (80) lorsque ce dernier est déplacé du premier état au deuxième état.

9. Dispositif de cathéter selon la revendication 4 ou selon l'une des revendications 5 à 8 si l'on se réfère à la revendication 4, dans lequel l'élément de verrouillage (80) comprend une ouverture (802) ayant une partie étroite (802a) et une partie adjacente plus large (802b), et dans lequel la tige (81) comprend une partie distale comprenant une section étroite (81b) ayant un diamètre qui est plus petit qu'un diamètre d'une partie proximale adjacente de la tige (81) de sorte que la tige (81) forme une étape (81c), Dans lequel, lorsque l'élément de verrouillage (80) est dans le premier état, ladite section étroite (81b) de la tige (81) est disposée dans la partie étroite (802a) de l'ouverture (802) et la tige (81) est configurée pour être pressée par le transporteur (76) contre l'élément de verrouillage (80) avec ladite marche (81c) afin de limiter le mouvement du transporteur (76) dans la direction distale (D).

10. Le dispositif de cathéter selon les revendications 4 et 9, dans lequel lorsque l'élément de verrouillage (80) est déplacé du premier état au second état, la tige (81) est disposée dans la partie large (802b) de l'ouverture (802) de sorte que la marche (81c) est autorisée à se déplacer à travers la partie large (802b) de l'ouverture (802) de la tige (81) par l'action du premier élément de ressort (760).

11. Dispositif de cathéter selon les revendications 4, 6 et 8, et selon la revendication 9 ou 10, dans lequel, lorsque le second élément élastique (810) est précontraint, le déplacement du dispositif de déplacement (76) et donc de la capsule (40) dans la direction proximale (P) entraîne le déplacement du second élément élastique (810) de la tige (81) dans la direction proximale (P), également, de sorte que ladite section étroite (81b) de la tige (81) se déplace dans la partie large (802b) de l'ouverture (802) de l'élément de verrouillage (802) à une certaine position du dispositif de déplacement (76), de sorte que ladite section étroite (81b) de la tige (81) se déplace dans la partie large (802b) de l'ouverture (802) de l'élément de verrouillage (80) à une certaine position du dispositif de déplacement (76), de sorte que le troisième élément à ressort (801) ramène l'élément de verrouillage (80) dans le premier état dans lequel ladite section étroite (81b) de la tige (81) est disposée dans la partie étroite (802a) de l'ouverture (802) de l'élément de verrouillage (80).

12. Dispositif de cathéter selon la revendication 6 ou selon l'une des revendications 7 à 11 si l'on se réfère à la revendication 6, dans lequel le bouton de déploiement (72) est supporté de manière rotative sur l'élément de guidage (75) de la poignée (70).
